Europäisches Patentamt

European Patent Office

Office européen des brevets

(11) Publication number: **0 378 223 B1**

**EUROPEAN PATENT SPECIFICATION**

(45) Date of publication of patent specification: **06.04.94**   (51) Int. Cl.⁵: **C12P 13/10**, //(C12P13/10, C12R1:13,1:15)

(21) Application number: **90100548.8**

(22) Date of filing: **11.01.90**

(54) **Process for producing L-arginine by fermentation.**

(30) Priority: **12.01.89 JP 5770/89**

(43) Date of publication of application:
**18.07.90 Bulletin 90/29**

(45) Publication of the grant of the patent:
**06.04.94 Bulletin 94/14**

(84) Designated Contracting States:
**BE DE FR GB IT**

(56) References cited:
**GB-A- 2 084 566**

**CHEMICAL ABSTRACTS, vol. 103, no. 19, November 1985, Columbus, OH (US); p. 585, no. 159100z&NUM;**

(73) Proprietor: **AJINOMOTO CO., INC.**
**5-8, Kyobashi 1-chome, Chuo-ku**
**Tokyo 104(JP)**

(72) Inventor: **Tsuchida, Takayasu, c/o Kawasaki Plant, Ajinomoto**
**Co. Inc., No. 1-1, Suzuki-cho, Kawasaki-ku**
**Kawasaki-shi, Kanagawa-ken(JP)**
Inventor: **Ohtsuka, Noboru, c/o Kawasaki Plant, Ajinomoto**
**Co. Inc., No. 1-1, Suzuki-cho, Kawasaki-ku**
**Kawasaki-shi, Kanagawa-ken(JP)**
Inventor: **Takeuchi, Hiroshi, c/o Kawasaki Plant, Ajinomoto**
**Co. Inc., No. 1-1, Suzuki-cho, Kawasaki-ku**
**Kawasaki-shi, Kanagawa-ken(JP)**
Inventor: **Uchibori, Haruo, c/o Kawasaki Plant, Ajinomoto**
**Co. Inc., No. 1-1, Suzuki-cho, Kawasaki-ku**
**Kawasaki-shi, Kanagawa-ken(JP)**

(74) Representative: **Strehl Schübel-Hopf Groening & Partner**
**Maximilianstrasse 54**
**D-80538 München (DE)**

## Description

L-Arginine is an important component of drugs for stimulating liver functions, amino acid infusion, total amino acid preparations, etc. The present invention relates to an improved process for producing L-arginine by fermentation.

In order to impart L-arginine productivity to microorganisms belonging to the genus Brevibacterium or the genus Corynebacterium it is known to provide the microorganisms with resistance to 2-thiazolealanine (hereafter simply referred to as 2-TA), arginine hydroxamate, etc. It is also known that the productivity of L-arginine is improved by imparting resistance to sulfa drugs or argininol resistance, resistance to chemicals such as 8-azaguanine, $\alpha$-amino-$\beta$-hydroxyvaleric acid, etc., in addition to the chemical resistance described above, and by imparting auxotrophy for amino acids such as L-histidine, L-proline, L-threonine, L-tryptophan, L-lysine, etc.

In order to reduce the production costs of L-arginine, it is important to improve the fermentation yield.

The problem to be solved by the present invention is to provide a fermentation process which allows the production of L-arginine in higher yields.

As a result of investigations to improve known microorganisms belonging to the genus Brevibacterium or the genus Corynebacterium and capable of producing L-arginine and to find strains causing a further improved fermentation yield, it has been found that strains capable of producing L-arginine in a higher yield than conventional L-arginine-producing strains are found among strains having resistance to X-guanidine (wherein X is an aliphatic chain or an aliphatic chain derivative) (hereafter simply referred to as X-GN).

The present invention relates to a process for producing L-arginine which comprises culturing an L-arginine-producing microorganism belonging to the genus Brevibacterium or the genus Corynebacterium in a liquid culture medium, producing and accumulating L-arginine in the culture medium and collecting L-arginine from the culture medium, wherein an L-arginine-producing microorganism is used, which has resistance to X-guanidine, wherein X is an aliphatic chain or an aliphatic chain derivative.

The microorganims used in the present invention are variants belonging to the genus Brevibacterium or the genus Corynebacterium, capable of producing L-arginine and having X-GN resistance. To obtain the variants of the present invention, L-arginine productivity may previously be imparted to wild strains described below and X-GN resistance may then be imparted thereto; alternatively , X-GN resistance may be first imparted and L-arginine productivity may then be imparted.

The compounds of the formula X-GN are guanidines having aliphatic chains, such as butenylguanidine, hexylguanidine, octylguanidine, etc. and derivatives of guanidines having aliphatic chains such as 4-methyl-3-butenylguanidine, etc.

Wild strains which can be parent strains of the variants of the present invention are known as bacteria belonging to the genus Brevibacterium or the genus Corynebacterium, especially as Coryneform L-glutamic acid producing bacteria and are exemplified by the following bacteria.

**Brevibacterium divaricatum ATCC 14020**
**Brevibacterium flavum ATCC 14067**
**Brevibacterium lactofermentum ATCC 13869**
**Brevibacterium saccharolyticum ATCC 14066**
**Corynebacterium acetoacidophilum ATCC 13870**
**Corynebacterium glutamicum ATCC 13032**

For mutation to produce variants of the present invention from these parent strains, a conventional method of mutation which comprises contacting these strains with N-methyl-N′-nitro-N-nitrosoguanidine, etc. can be appropriately used. The isolation of the variant of the present invention can be effected by collecting strains which can grow in a medium containing X-GN.

A concrete mutation method to produce the variant of the present invention and the relationship between the concentration of octylguanidine (hereafter simply referred to as O-GN) as one of the compounds X-GN and the growth of the strain are shown below.

Method of mutation

Bacteria cells of Brevibacterium flavum AJ 11169, FERM P-4161 (2-TA resistant strain derived from ATCC 14067) and Corynebacterium glutamicum AJ 12092, FERM P-7273 (2-TA resistant strain derived from ATCC 13032), which had been grown on bouillon agar slants at 30°C for 24 hours, were suspended in M/30 phosphate buffer solution in a cell density of $10^8$ to $10^9$/ml. To the cell suspension were added 500 μg/ml of N-methyl-N′-nitro-N-nitrosoguanidine. The mixture was maintained at 30°C for 20 minutes. Then the cells were collected by centrifugation. After thoroughly washing with M/30 phosphate buffer solution, the cells were inoculated on a medium having the following composition and cultured at 31.5°C for 4 to 10 days.

| Composition of Medium (pH 7.0) | |
|---|---|
| Component | Content |
| Glucose | 1.0 g/dl |
| Urea | 0.2 g/dl |
| $KH_2PO_4$ | 0.1 g/dl |
| $MgSO_4.7H_2O$ | 0.1 g/dl |
| $FeSO_4.7H_2O$ | 0.002 g/dl |
| $MnSO_4.7H_2O$ | 0.002 g/dl |
| Biotin | 100 $\mu$g/l |
| Thiamine hydrochloride | 100 $\mu$g/l |
| O-GN | 0.1 g/dl |
| Agar | 2.0 g/dl |
| (O-GN = Octylguanidine) | |

From the strains grown in the agar medium, Brevibacterium flavum AJ 12429, FERM BP-2227(2-TA resistant, O-GN resistant) and Corynebacterium glutamicum AJ 12430, FERM BP-2228(2-TA resistant, O-GN resistant) were obtained as having high productivity of L-arginine.

The O-GN resistance of the thus obtained variants was compared with that of the parent strains.

Onto a medium composed of 0.5 g/dl of glucose, 0.15 g/dl of urea, 0.15 g/dl of ammonium sulfate, 0.3 g/dl of $KH_2PO_4$ 0.1g/dl of $K_2HPO_4$, 0.01 g/dl of $MgSO_4.7H_2O$, 0.1 mg/dl of $CaCl_2.2H_2O$, 100 $\mu$g/l of biotin, 100 $\mu$g/l of thiamine hydrochloride, 0.002 g/dl of $FeSO_4.7H_2O$, 0.002 g/dl of $MnSO_4.7H_2O$ and O-GN in the amount shown in the table and adjusted to pH of 7.0, there was inoculated a suspension of the cells in sterile water, which were obtained by culturing on a slant of natural medium (1 g/dl of peptone, 1 g/dl of yeast extract and 0.5 g/dl of NaCl, pH 7.0) for 24 hours. After culturing for 24 hours, the growth degree was determined in terms of turbidity.

## Table 1

| Inhibitor<br>Strain | Concentration of O-GN ($\mu$g/ml) | | | | |
|---|---|---|---|---|---|
| | 0 | 25 | 50 | 100 | 200 |
| Brevibacterium flavum AJ 11169 (FERM P-4161) | 0.90 | 0.50 | 0.10 | 0 | 0 |
| Brevibacterium flavum AJ 12429 (FERM BP-2227) | 0.85 | 0.85 | 0.70 | 0.30 | 0 |
| Corynebacterium glutamicum AJ 12092 (FERM P-7273) | 0.95 | 0.70 | 0.15 | 0 | 0 |
| Corynebacterium glutamicum AJ 12430 (FERM BP-2228) | 0.95 | 0.96 | 0.90 | 0.60 | 0.10 |

In many cases, the yield increases by further imparting to the aforesaid variants properties already known to improve the productivity of L-arginine, such as sulfaguanidine resistance, argininol resistance or 8-azaguanine resistance.

Media used for culturing such variants are conventional media containing carbon sources, nitrogen sources, inorganic ions, substances satisfying the auxotrophy described above and, if necessary, other organic trace nutrients including vitamins, etc.

As carbon sources, there are preferably used carbohydrates such as glucose, sucrose, etc., organic acids such as acetic acid, etc. As nitrogen sources, there are preferably used ammonia water, ammonia

3

gas, ammonium salts, etc. As inorganic ions, potassium ions, sodium ions, magnesium ions, phosphate ions, and the like are appropriately added to media, depending upon necessity.

The incubation is preferably conducted under aerobic conditions. When the incubation is carried out while adjusting the pH of the medium to a range from 4 to 8 at a temperature of from 25°C to 37°C, more preferable results can be obtained. Thus, during a culture period of 1 to 7 days, remarkable amounts of L-arginine are produced and accumulated in the media.

For collecting L-arginine from the culture solution, conventional methods such as a method using an ion exchange resin, etc. can be used.

The present invention is further described with reference to the following example.

## Example

A medium containing 10 g/dl of glucose, 7 g/dl of $(NH_4)_2SO_4$, 0.1 g/dl of $KH_2PO_4$, 0.04 g/dl of $MgSO_4.7H_2O$, 1 mg/dl of $FeSO_4.7H_2O$, 1 mg/dl of $MnSO_4.4H_2O$, 100 $\mu$g/l of thiamine.HCl, 100 $\mu$g/l of biotin, 60 mg of soybean protein acid hydrolysate (calculated as total nitrogen) and 5 g/dl of calcium carbonate (separately sterilized) was adjusted to pH 7.0 and 20 ml of the medium was charged into a 500 ml flask with a shoulder followed by sterilization by heating. One platinum loop of the strain shown in Table 1 was inoculated on the medium and cultured while shaken for 4 days at 31.5°C. L-Arginine was produced and accumulated in the culture solution of each strain in the amount shown in Table 2.

Table 2

|  | Property | Amount of L-Arginine Accumulated (g/dl) |
|---|---|---|
| Brevibacterium flavum AJ 11169 (FERM P-4161) | 2-TA$^r$ | 1.9 |
| Brevibacterium flavum AJ 12429 (FERM BP-2227) | 2-TA$^r$.O-GN$^r$ | 3.6 |
| Corynebacterium glutamicum AJ 12092 (FERM P-7273) | 2-TA$^r$ | 1.7 |
| Corynebacterium glutamicum AJ 12430 (FERM BP-2228) | 2-TA$^r$.O-GN$^r$ | 3.4 |
| 2-TA$^r$ : 2-thiazolealanine resistance O-GN$^r$ : octylguanidine resistance | | |

AJ 12429 was cultured by the method described above to give 1 liter of the culture solution. The culture solution was centrifuged to remove the cells and the like. The supernatant was passed through a weakly acidic ion exchange resin "Amberlite" C-50 (NH$_4$ type). After washing the resin with water, L-arginine was eluted with 2 N NH$_4$OH and the eluate was concentrated. From the concentrate, 19.5 g of L-arginine was obtained as crude crystals.

## Claims

**1.** A process for producing L-arginine which comprises culturing an L-arginine-producing microorganism belonging to the genus Brevibacterium or the genus Corynebacterium in a liquid culture medium, producing and accumulating L-arginine in the culture medium and collecting L-arginine from the culture medium, characterised in that said L-arginine-producing microorganism has resistance to X-guanidine, wherein X is an aliphatic chain or an aliphatic chain derivative.

## Patentansprüche

**1.** Verfahren zur Herstellung von L-Arginin, welches das Züchten eines L-Arginin produzierenden Mikroorganismus des Genus Brevibacterium oder des Genus Corynebacterium in einem flüssigen Kulturmedium, die Bildung und Anreicherung von L-Arginin in dem Kulturmedium und die Gewinnung von L-Arginin aus dem Kulturmedium umfaßt, dadurch gekennzeichnet, daß der L-Arginin produzierende Mikroorganismus Resistenz gegen X-Guanidin hat, worin X eine aliphatische Kette oder ein Derivat einer aliphatischen Kette ist.

**Revendications**

1.  Procédé pour la préparation de L-arginine lequel comprend la culture de microorganismes produisant de la L-arginine et appartenant au genre Brevibacterium ou au genre Corynebacterium dans un milieu de culture liquide, la production et l'accumulation de L-arginine dans le milieu de culture et le recueil de L-arginine à partir du milieu de culture, caractérisé en ce que lesdits microorganismes produisant la L-arginine sont résistants à la X-guanidine, dans laquelle X représente une chaîne aliphatique ou un dérivé de chaîne aliphatique.